(19) **Europäisches Patentamt**
**European Patent Office**
**Office européen des brevets**

(11) **EP 3 064 134 B1**

(12) **EUROPEAN PATENT SPECIFICATION**

(45) Date of publication and mention
of the grant of the patent:
**03.04.2019 Bulletin 2019/14**

(51) Int Cl.:
***A61B 5/11*** *(2006.01)*

(21) Application number: **16158760.5**

(22) Date of filing: **04.03.2016**

(54) **INERTIAL MOTION CAPTURE CALIBRATION**

KALIBRIERUNG ZUR ERFASSUNG VON TRÄGHEITSBEWEGUNG

ÉTALONNAGE DE CAPTURE DE MOUVEMENT INERTIEL

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **05.03.2015 US 201514639776**

(43) Date of publication of application:
**07.09.2016 Bulletin 2016/36**

(73) Proprietor: **Xsens Holding B.V.
7521 PR Enschede (NL)**

(72) Inventors:
• **Hol, Jeroen D.
7552 NC Hengelo (NL)**
• **Bellusci, Giovanni
7511 AM Enschede (NL)**

(74) Representative: **V.O.
P.O. Box 87930
Carnegieplein 5
2508 DH Den Haag (NL)**

(56) References cited:
**US-A1- 2008 091 373     US-A1- 2011 028 865
US-A1- 2014 303 524     US-B2- 7 725 279**

• **SCHMITZ ANDREAS ET AL: "Learning Footstep
Prediction from Motion Capture", 2011,
NETWORK AND PARALLEL COMPUTING;
[LECTURE NOTES IN COMPUTER SCIENCE;
LECT.NOTES COMPUTER], SPRINGER
INTERNATIONAL PUBLISHING, CHAM, PAGE(S)
97 - 108, XP047432372, ISSN: 0302-9743 ISBN:
978-3-642-24784-2**

**Description**

TECHNICAL FIELD

The present disclosure is related generally to inertial motion capture and, more particularly, to a system and method for inertial motion capture calibration.

BACKGROUND

It is frequently desired to determine the position of an object, such as a golf club, or of a person, or of a body part such as a golfer's hand, forearm, and so on. Indeed, the uses for motion sensing systems include sports analysis, medical analysis, animation, entertainment, virtual reality and other uses. For such purposes, one or more motion sensors are typically placed on each object or part of interest, and the data from such sensors is collected and processed to provide an indication of position, velocity, acceleration, orientation or other parameters.

Such sensors are typically self-referenced, meaning they provide position or motion data without reference to an external component such as a satellite. Inertial sensors are often used where self-referenced sensors are needed. These sensors operate via internal gyroscopes and accelerometers. While inertial sensor systems can be very accurate, the sensors detect changes in orientation and velocity, rather than sensing orientation, velocity or position directly. As such, there is a need to determine the initial spatial state of the sensor before delta readings can be processed to provide accurate velocity, position and orientation information.

In greater detail, with respect to capturing human motion, inertial motion capture technology works by integrating 3D gyroscope and 3D accelerometer signals to generate orientation and position for each tracked object or human body segment. This is done while ensuring that the calculated orientation and position values remain consistent with any physical constraints, such as, with respect to a human body, the constraint that the body segments remain linked by their joints.

However, in order to achieve good motion tracking performance it is important that the mounting of the sensing unit on each segment is known, both in terms of orientation, typically called sensor-to-segment alignment, and in terms of position, commonly expressed as lever arms. Existing methods used to determine said mounting parameters suffer from severe drawbacks.

For example, the sensor-to-segment alignment method described in US7725279B2 requires that the subject stands motionless in a predefined position, in such a way that the orientation of the segments in a reference frame is known. From stationary accelerometer and magnetometer measurements, the orientation of every sensor is calculated. Taking the difference between the sensor orientation and the segment orientation now gives the sought sensor-to-segment alignment.

The major limitation of this approach lies in the requirement for a homogenous magnetic field at all the sensor positions. When this requirement is not satisfied, the calculated sensor orientations do not share a common reference frame which introduces errors in the sensor-to-segment calibration. While in some cases this might not pose significant limitations for practical use of inertial motion capture, there are many scenarios where this approach cannot be used. Examples include indoor usage, where steel of the construction is known to locally distort the magnetic field by several tens of degrees easily, or usage near medical devices such as prostheses, where moving parts of the construction or actuation currents result in magnetic distortions. Furthermore, if the subject does not assume the exact prescribed posture for any reason, the method gives poor performance.

US7725279B2 also describes a method wherein values for the lever arms are derived from a biomechanical scaling model which provides the length of individual segments, in combination with a predefined sensor mounting, for instance by having the subject wearing a suit. The results of this approach are generally inaccurate and not subject specific. Hence, they result in degraded inertial motion capture performance, which may manifests as incorrect step length, unrealistic motion or physically impossible poses with intersecting limbs.

An alternative method described in the aforementioned patent is to estimate the lever arms from the instantaneous acceleration of a joint connecting two adjoining segments: the measured acceleration vectors from adjoining segments are translated to the joint center and expressed in a common frame, giving equality since the segments cannot separate. Instead of comparing acceleration vectors, which requires known (relative) orientation to express them in a common frame, it is also possible to equate their magnitudes. From a dataset with enough excitation of the segments, the lever arms can be estimated by enforcing either equality to hold for all measurements.

The main limitation of this approach is that the translation of the instantaneous acceleration measurements to the joint centers, which is done using the well-known relation

$$a_{joint} = a_{sensor} + \dot{\omega} \times r + \omega \times \omega \times r,$$

requires the instantaneous angular acceleration $\dot{\omega}$. The instantaneous angular acceleration however is not measured directly; rather, it is derived from the angular velocity measurements of the gyroscopes. Due to its volatile nature and large ranges (a human can easily achieve angular acceleration values over 50000 deg/s for durations up to several ms), this puts stringent requirements in terms of large bandwidths combined with high sampling rates on both the accelerometers and the gyroscopes. These requirements are not feasible in many applications, including low-power, always on, wireless sensing devices.

Additionally, in many applications it is common to pre-process the signals with SDI as in US2011109438A1 in order to reduce the output rate by converting high-rate angular velocity and acceleration into lower rate orientation increments and velocity increments. In this way, however, instantaneous acceleration and instantaneous angular velocity are lost, which renders these signals useless for lever arm estimation using the approaches previously proposed in art based on translation of instantaneous acceleration.

The present disclosure is directed to systems and methods that can eliminate some of the shortcomings noted in this Background section. However, it should be appreciated that any such benefit is neither a limitation on the scope of the disclosed principles nor of the attached claims, except to the extent expressly noted in the claims. Additionally, the discussion of technology in this Background section is reflective of the inventors' own observations, considerations, and thoughts, and is in no way intended to accurately catalog or comprehensively summarize the prior art. As such, the inventors expressly disclaim this section as admitted or assumed prior art with respect to the discussed details, other than for the existence of the references noted by patent number or application number. For these, the reader is urged to read the relevant reference in its entirety for a full and accurate understanding of that art.

SUMMARY

In this disclosure, a method for inertial motion capture calibration is disclosed which does not rely on the magnetic field to estimate the sensor-to-segment alignment. Additionally, a method for calibration of lever arms is disclosed which does not require high output rate inertial measurements to work. The two methods can be combined into a simultaneous, subject specific, calibration.

According to the invention, the disclosed principles provide a method of inertial motion capture calibration according to claim 1.

The subject may be a human body, with limbs, appendages, and so on connected by joints. A preferred embodiment is provided according to claim 10.

Moreover, the 3D accelerometer and 3D gyroscope data may comprise orientation increment and velocity increment signals obtained from pre-processing with SDI (strap down integration).

BRIEF DESCRIPTION OF THE SEVERAL VIEWS OF THE DRAWINGS

While the appended claims set forth the features of the present techniques with particularity, these techniques, together with their objects and advantages, may be best understood from the following detailed description taken in conjunction with the accompanying drawings of which:

Figure 1 is a schematic illustration of a human leg consisting of three segments and two joints, with each segment having at least one sensing unit thereon in accordance with an embodiment of the described principles;

Figure 2 is a usage representation showing stride error;

Figure 3 is a modular view of an example system for implementing various embodiments of the disclosed principles;

Figure 4 is a flowchart showing a calibration process of estimating unknown 3D position vectors in accordance with an embodiment of the disclosed principles;

Figure 5 is a data plot showing lever arm estimation results for SDI inputs using an embodiment of the disclosed principles and a comparison technique;

Figure 6A is a usage representation showing sensor-to-segment calibration results in accordance with an embodiment of the disclosed principles, with a transparent reference overlay;

Figure 6B is another usage representation showing sensor-to-segment calibration results in accordance with an embodiment of the disclosed principles, with a transparent reference overlay;

Figure 7 is a knee joint angle plot showing knee flexion and extension results in accordance with an embodiment of the disclosed principles;

Figure 8A is a usage schematic showing a pose estimate during normal walk, before calibration in accordance with an embodiment of the disclosed principles, with a transparent reference overlay;

Figure 8B is a usage schematic showing a pose estimate during normal walk, after calibration in accordance with an embodiment of the disclosed principles, with a transparent reference overlay; and

Figure 9 is a flowchart showing a process of estimating unknown 3D orientations using segment orientation constraints in accordance with an embodiment of the disclosed principles.

DETAILED DESCRIPTION

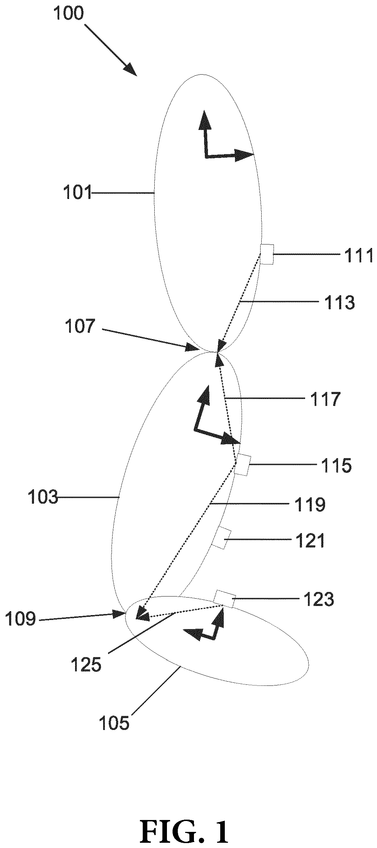

Turning now to a more detailed discussion in conjunction with the attached figures, Figure 1 is a schematic illustration of a human leg consisting of three segments and two joints, with each segment having at least one sensing unit thereon in accordance with an embodiment of the described principles. More generally, for purposes of inertial motion capture, the body of the subject, or part of it, is assumed to consist of N segments that are linked to each other using joints. Additionally, each segment is instrumented with at least one sensing unit, containing at least a 3D gyroscope and a 3D accelerometer.

In the illustrated figure, the multi-segment object of interest 100 is a human leg and foot. This object of interest 100 is shown as three segments 101, 103 and 105, with segment 101 being the upper leg, segment 103 being the lower leg, and segment 105 being the foot. A first joint 107 (e.g., the knee) joins segment 101 with segment 103, and a second joint 109 (e.g., the ankle) joins segment 103 with segment 105. The joints 107 and 109 may be modeled as point pivots or, in alternative embodiment, without limiting the applicability of the method, as hinge joints or ball-in-socket joints.

In the illustrated embodiment, the upper leg segment 101 is instrumented with a first inertial sensing unit 111. The manner of affixing the first inertial sensing unit 111 to the segment 101 is not critical, but should be done in a manner that minimizes relative movement between the first inertial sensing unit 111 and the underlying skeletal structure. Various methods of affixing the first inertial sensing unit 111 to the first segment 101 include affixing with an adhesive, affixing with a band or strap, affixing via a pocket in a garment and so on. The first inertial sensing unit 111 has a lever arm 113 to the first joint 107.

In the illustrated embodiment, the lower leg segment 103 is instrumented with a second inertial sensing unit 115. The manner of affixing the second inertial sensing unit 115 to the segment 103 may be by any of the means discussed above or otherwise. The second inertial sensing unit 115 has a lever arm 117 to the first joint 107 and a lever arm 119 to the second joint 109.

A third inertial sensing unit 121 is shown affixed to the lower leg segment 103 as well. This is simply to illustrate that more than one inertial sensing unit may be used for a given segment.

Finally, the foot segment 105 is instrumented with a fourth inertial sensing unit 123. The manner of affixing the fourth inertial sensing unit 123 to the segment 105 may be by any of the means discussed above or otherwise. The fourth inertial sensing unit 123 has a lever arm 125 to the second joint 109.

To estimate the motion of the subject instrumented as in Figure 1, the 3D gyroscope and 3D accelerometer signals of each inertial sensor unit are integrated to generate orientation and position values respectively, for each sensing unit, while ensuring that the orientation and position values for the segments are consistent with the physical joint constraints.

In other words, the orientation and position values for each segment must be such that as a whole, the jointed model remains intact at the joints. The lever arms between sensing units and joints are used, in an embodiment, to link the segments (that is, to ensure that the 3D joint centers from different segments at the same joint lie at the same point).

The estimated motion of the subject will inevitably drift in both position and orientation due to error integration and the absence of absolute orientation and position aiding sources. However, the individual sensor kinematics are constrained and coupled by the joints. In particular, under mild conditions of motion, e.g. an acceleration caused by sitting in an accelerating car or just walking, all relative sensor orientations become observable and can be accurately calculated.

An intuitive explanation for this property is the following: if one fixes the initial condition on orientation of one sensing unit, this results in a certain position trajectory of the sensing unit and, assuming known lever arms, a certain trajectory of its joint centers. The latter imposes in turn a relative position aiding for the adjoining sensing units. From GPS literature it is well known that under non-constant acceleration, position aiding results in completely observable sensor orientation. Hence, conditioned onto the orientation of the first sensing unit, the orientation of the adjoining sensing unit becomes observable, which is what is implied by observable relative sensor orientation. In other words, the orientation of all sensor units with respect to a common, though drifting frame can be consistently estimated without drift.

Note that this observability of relative orientation is obtained without relying on magnetometers to stabilize the orientation of every sensing unit, in particular the heading components. Moreover, the inertial motion capture system is formulated using the orientations of the sensor units only; the orientations of the segments are not part of it.

With this in mind, we turn to the question of lever arm calibration. It is important to note that the inertial motion capture problem previously described in detail contains redundant sources of information. Note that this redundancy results from the specific nature of the problem addressed; in other words, the use of a linked segment model wherein each segment includes an attached sensing unit results in redundant available information.

When the lever arms between sensing unit and corresponding segment origin are not a properly calibrated, this redundancy of available information may result in inconsistencies. However, these inconsistencies are easily seen when calculating specific motion tracking quantities, e.g., when calculating orientation and position trajectories of the sensing units. The quantities under discussion are not always relevant for the final application, which, for example, might require orientation and position trajectories of the body segments instead.

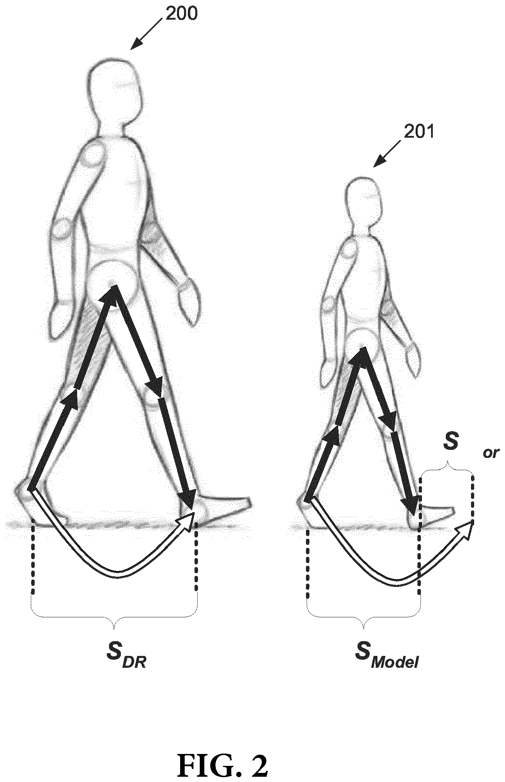

An example more clearly demonstrates the foregoing observation. Consider a subject 200, instrumented as above and walking. This situation is shown in Figure 2. In this example, the information of interest is the length $S$ of the subject's stride. During walking, the subject's stride length $S$ may be determined by dead-reckoning based on the data from the sensing unit on the subject's foot 105. This results in a first value of $S= S_{DR}$.

Alternatively, the subject's stride length can be independently determined from a vector sum of lever arms between sensing units and joints. The stride length $S_{Model}$ calculated in this way should be consistent with the stride length $S_{DR}$ calculated via dead-reckoning. However, if the lever arms are incorrect, as shown in the second rendering of the subject 201, these methods will not yield the same stride length. Indeed, as seen in the figure, the use of shorter-than-actual lever arms will result in a shorter-than-actual stride length and a mismatch $S_{Error}$ between the accurate stride length $S= S_{DR}$ and the erroneous stride length $S_{Model}$.

Following this observation, instead of treating the lever arms as constants with an *a priori* determined value, as is traditionally done, the method according to the described principles instead formulates a motion tracking problem in a way suited specifically to minimize this source of inconsistency. To this end, unknown 3D position vectors are introduced between each sensing unit and the joint centers of corresponding body segments, and the unknown 3D position vectors are estimated together with the sensing unit position and orientation trajectories over time.

In contrast to certain prior techniques, such as those described in US7725279, no translation of acceleration is needed; as such, the techniques described herein do not suffer from many of the limitations and problems of current techniques.

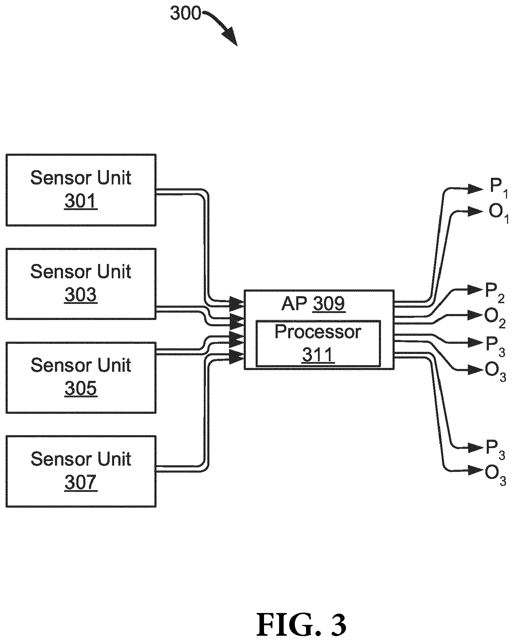

Before describing the example technique in further detail, a modular diagram of the sensor system is given to provide reference for the steps of the method. Thus, Figure 3 shows an example configuration for a sensor system 300 for use with respect to embodiments of the described principles.

In the illustrated example, the sensor system includes a plurality of inertial measurement units (IMUs) 301, 303, 305, 307, which are sometimes also referred to as inertial sensor units. Each IMU 301, 303, 305, 307 generates a signal indicative of a sensed 3D angular velocity (change in orientation) and a signal indicative of a 3D acceleration (change in velocity). The angular velocity and the acceleration may be positive, zero or negative with a defined sensor range for each sensor type.

Each IMU 301, 303, 305, 307 provides the signals indicative of angular velocity and acceleration to a master unit or application processor (AP) 309. The AP 309 is a processor-driven unit (see processor 311) capable of managing data intake and output and of executing data manipulations such as integration and other manipulations that are discussed elsewhere herein.

Having received a signal indicative of change in orientation and a signal indicative acceleration from each of the IMUs 301, 303, 305, 307, the AP 309 converts the received data into an output consisting of multiple pairs of orientation and position estimates $(O_n, P_n)$, one pair for each IMU 301, 303, 305, 307. It will be appreciated that an inertial motion tracking system may be implemented with a different architecture and/or a different number of IMUs 301, 303, 305, 307 than shown in Figure 3. Moreover, those of skill in the art will be aware that the motion tracking process operations may take place at a single entity such as the AP 309 or across multiple entities, and may take place at the subject or remotely.

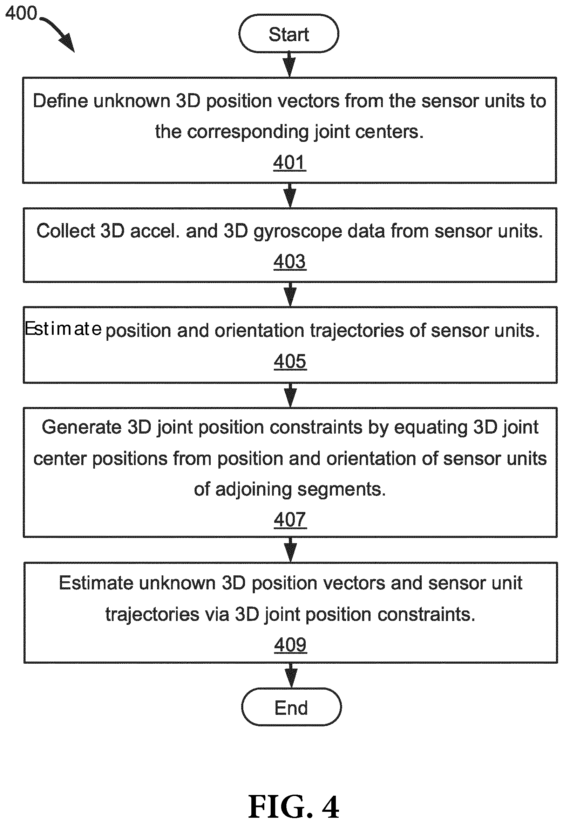

Although the described principles may be implemented in a variety of ways, an example process for lever arm calibration in accordance with an embodiment of the described principles is shown in Figure 4. In particular, Figure 4 shows a process 400 that estimates position vectors and trajectories using joint constraints in a multi-segment body of interest.

At stage 401 of the process 400, the processor 311 defines unknown 3D position vectors from the inertial sensing units 301, 303, 305, 307 to the corresponding joint centers. The processor 311 then collects 3D accelerometer and 3D gyroscope data from the sensing units 301, 303, 305, 307 at stage 403 and estimates position and orientation trajectories of the sensing units 301, 303, 305, 307 at stage 405 over time.

Subsequently at stage 407, the processor 311 generates 3D joint position constraints by equating the 3D joint center positions derived from the position and orientation of two sensing units attached to adjoining segments. Finally at stage 409, the processor 311 estimates the unknown 3D position vectors and the sensing unit trajectories by applying the 3D joint position constraints.

It is noted that the described lever arm calibration method uses the 3D accelerometer and 3D gyroscope signals for integration to position and orientation. Therefore, although generally applicable to the case in which angular velocity and acceleration signals are available, the method is especially well suited to accept signals pre-processed with SDI (strap down integration), i.e., orientation increments and velocity increments. Indeed, SDI reduces the output rate by retaining integral quantities such as position, velocity and orientation. However, SDI sacrifices the instantaneous angular velocity and acceleration signals which are required by the prior art; when SDI is performed, said instantaneous quantities can no longer be retrieved.

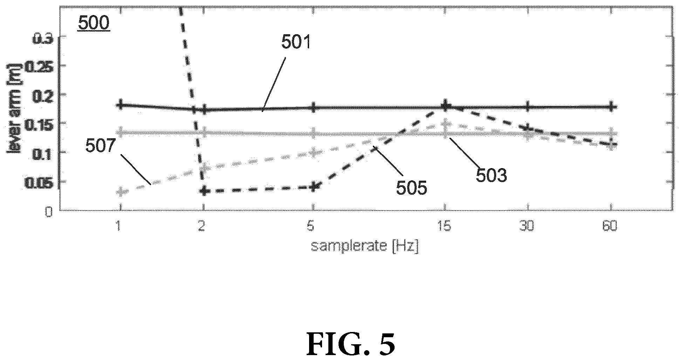

The performance of the method described herein is illustrated graphically in Figure 5, which shows lever arm estimation results for the knee joint on a normal walking trial using SDI preprocessed measurements, as is common practice for commercially available wireless sensing units. The plot 500 includes four traces, namely the upper leg estimation 501 in accordance with the disclosed principles, the lower leg estimation 503 in accordance with the disclosed principles, the upper leg estimation 505 in accordance with the prior techniques, and the lower leg estimation 507 in accordance with the prior techniques.

It can be seen that the lever arm estimates calculated in accordance with the disclosed principles show consistent results independent of the used SDI rate (and are in line with ruler measurements of the measurement setup), whereas the prior method fails to give correct results, even for 60Hz data. Note that 60Hz is currently the maximum transmission rate of state-of-the-art commercially available full body inertial motion capture systems using wireless sensing units. Compared to the prior art, the compatibility with SDI opens new application areas including low-power, always on, wireless sensing devices since it allows a significant further reduction of the transmission rate resulting in improved performance with respect to wireless range or power consumption.

Using the described techniques, it is generally easy to observe the lever arms when small excitations in each degree of freedom of the joints are present. An intuitive explanation for this condition is that when two segments change orientation with respect to each other, their relative change in position will be coupled to the joint center position and hence to the lever arms. This in turn will allow the latter to be consistently observed. Note that for a (perfect) hinge joint, the joint center can lie anywhere on the (virtual) joint axis and hence the pair of lever arms are not uniquely defined. However, the (time varying) relative distance between the inertial sensing units remains observable in this case and hence motion capture performance is unaffected.

Those of skill in the art will appreciate that the described techniques can be extended to include other knowledge if available, e.g., a statistical scaling model of the human body, or data that a suit constrains sensing unit placement. The main benefit of doing so would be to increase robustness. This might be particularly beneficial in circumstances where there is insufficient excitation.

With respect to sensor-to-segment alignment calibration, in typical inertial motion tracking applications, the user is usually interested in body segment quantities rather than sensing unit quantities; for example 3D joint angles or segment orientation or position trajectories. For this reason, in order to translate sensor orientations to segment orientations, the sensor-to-segment alignments need to be determined. As described in previous sections, the prior art addresses this problem by relying on magnetometer readings to express the sensing unit orientations in a common frame, making this method not applicable in commonly occurring cases in which the magnetic field is not homogeneous.

The disclosed principles solve this problem by exploiting the fact that the sensing unit orientations can be tracked consistently over time without the specific alignment between sensing unit and corresponding body segment being known. This implies that adding additional information related to the segment orientations over time is sufficient to estimate the unknown sensor-to-segment alignments.

More specifically, since the sensing units' relative orientation trajectories are consistently tracked over time, a set of segment orientation constraints over time, either with respect to an external frame, or with respect to another segment frame, or with respect to a sensor frame, are sufficient to serve to the purpose. Since the number of unknowns which need to be solved are 3N, where N represents the number of sensing units attached to body segments, it is necessary to formulate at least 3N independent (one-dimensional) segment orientation constraints.

For the sake of reference, each of these (one-dimensional) segment orientation constraint can be more formally expressed as

$$f(R_t) = 0,$$

where the function $f: SO_3^n \to \mathbb{R}^1$ operates on a 3D segment orientation R at n (one or more) time instants t relative to another frame. As said, this other frame can be any of an external reference frame, a sensor frame, or another segment frame.

Segment orientation constraints can be derived from various sources or underlying physical principles, including, without limiting the applicability of the invention to these exemplary cases, specification of a segment orientation in an external frame at a given time instant, specification of a vector in two different segment frames or in a segment frame and in a sensor frame, specification of a joint angle pattern between two segments, etc.

To further illustrate the concept of independent segment orientation constraints, consider the case of a subject walking for a couple of gait cycles and then standing motionless in a known pose for a couple of seconds. The excitation during walking allows the orientations of the sensing units with respect to a common reference frame to be consistently estimated over time; this also holds during the motionless period.

At the same time, during the motionless period, the orientation of all *N* segments with respect to the reference frame ($R_{\text{known pose}}$) is known from the known pose. Combining both sources of information, the unknown sensor-to-segment alignments can be estimated for all segments. This can be more formally seen by observing that the set of segment orientation constraints during the motionless period can be formulated in this case as

$$RR_{\text{known pose}}^{-1} - I_{3\times3} = 0_{3\times3}$$

for every segment. Note, this results in 9 one-dimensional segment orientation constraints per each segment; of these constraints, 3 are independent. Hence, the set of segment orientation constraints meets the requirement of 3N independent constraints previously described. Note again that in contrast to prior art this approach does not use magnetometers and as such functions in any magnetic environment, including heavily distorted ones.

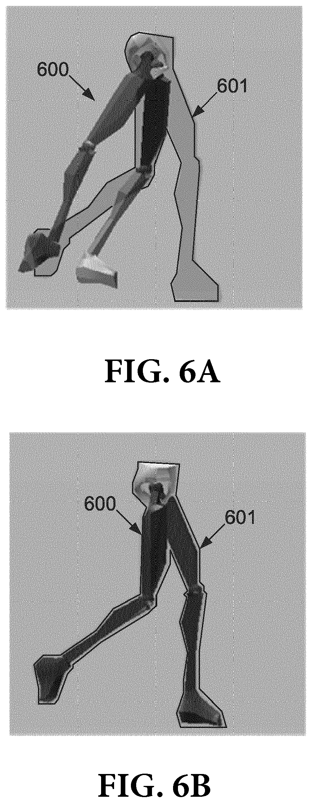

The performance of the disclosed sensor-to-segment calibration algorithm for this example is illustrated in Figures 6A and 6B. The figure 600 shows a pose estimate during normal walk, before (6A) and after (6B) calibration, including a transparent reference overlay 601 in Figure 6A (before) and Figure 6B (after). It can be seen that before calibration, the motion capture performance is very poor and the difference between the figure 600 and the reference overlay 601 is significant. This is a consequence of using incorrect sensor-to-segment alignments. After calibration (6B), accurate results are obtained since the sensor-to-segments have been correctly estimated.

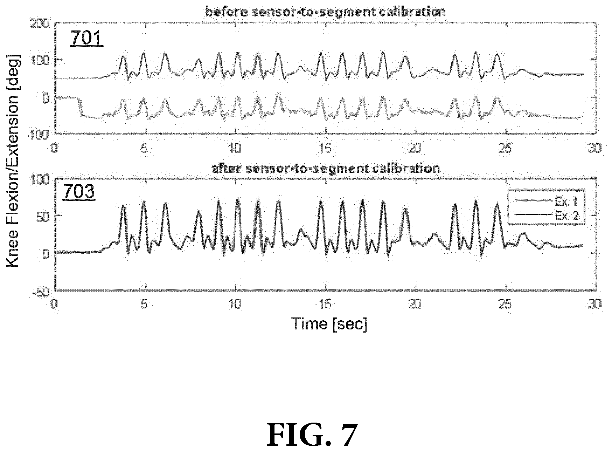

This behavior can also be seen in the joint angles labeled Ex. 1 in Figure 7. From the figure it can be seen that the knee flexion/extension before calibration (plot 701) is incorrect, since as shown in plot 703, it suffers from a large offset among other things. After calibration, a normal gait pattern can be observed in plot 703.

To further illustrate the concept of independent segment orientation constraints, consider the case of N = 2 segments, each with a sensing unit attached to it, and connected by a hinge joint, as for example a knee. Such a one dimensional joint defines an axis of rotation. From a dataset containing some excitation of the joint, the relative orientation of the sensors can be determined; this implies that the axis of rotation can be resolved in both sensor frames. In this way it is possible to introduce constraints which define the z-axis of each of the two segments ($v_{\text{z-axis}}$) to be the axis of rotation ($v_{\text{axis of rotation}}$). These constraints can be formulated as

$$Rv_{\text{axis of rotation}} - v_{\text{z-axis}} = 0_{2\times1}$$

for each segment. Note that this results in three one-dimensional segment orientation constraints per segment, of which only two constraints are independent.

To meet the independence criterion of 3N = 6 independent segment orientation constraints and obtain a complete sensor-to-segment definition, it is possible to further define the y-axis of both segments ($v_{\text{y-axis}}$) to be orthogonal to the joint lever arm ($v_{\text{lever arm}}$). This constraint can be formulated as:

$$v_{\text{y-axis}} Rv_{\text{lever arm}} = 0_{1\times1}$$

for each segment. Note that in contrast to prior art this approach does not depend on the magnetic environment, nor does it depend on the subject assuming a prescribed pose.

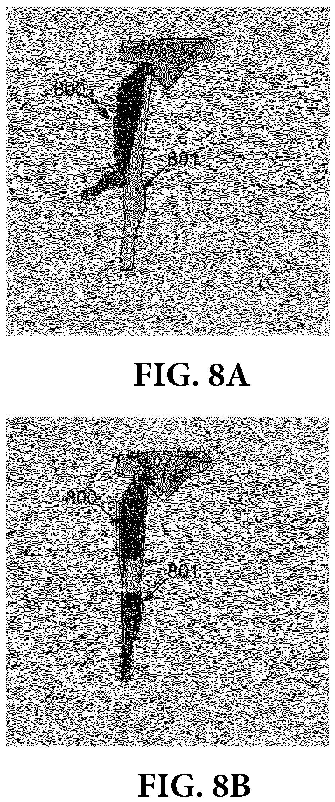

The performance of the proposed sensor-to-segment calibration algorithm for this example is illustrated in Figure 8. The figure shows a pose estimate 800 during normal walk, before (8A) and after calibration (8B), including a transparent reference overlay 801. It can be seen that before calibration, the motion capture performance is very poor. This is a consequence of using incorrect sensor-to-segment alignments. After calibration, accurate results are obtained since the sensor-to-segments have been correctly estimated. This behavior can also be seen in the joint angles, see the lines labeled Ex. 2 in Figure 7. After calibration (plot 703), a normal gait pattern can be observed, in contrast to before calibration (plot 701). Note that the calibrated results match those of the previous example.

It will be appreciated that alternative formulations of segment orientation constraints are possible. For example, the sensor-to-segment alignments of both segments can be defined by specifying the 3D joint angle between the segments to have a certain pattern in time. In case the joint has a single degree of freedom, as for example a hinge joint, a natural option is to impose two components of the joint angle be equal to zero.

$$I_{2\times3} \, \text{jointAngle}(R) = 0_{2\times1}$$

for at least two time instants with different poses. Due to the single degree of freedom of the joint, this results in a set segment orientation constraints with only four independent entries. To meet the independence criterion of 3N = 6 independent segment orientation constraints and obtain a complete sensor-to-segment definition, it is possible to additionally define the y-axis of each segment to be orthogonal to the joint lever arm, as discussed above. Although resulting in different types of segment orientation constraints, this alternative sensor-to-segment definition leads to similar calibration results.

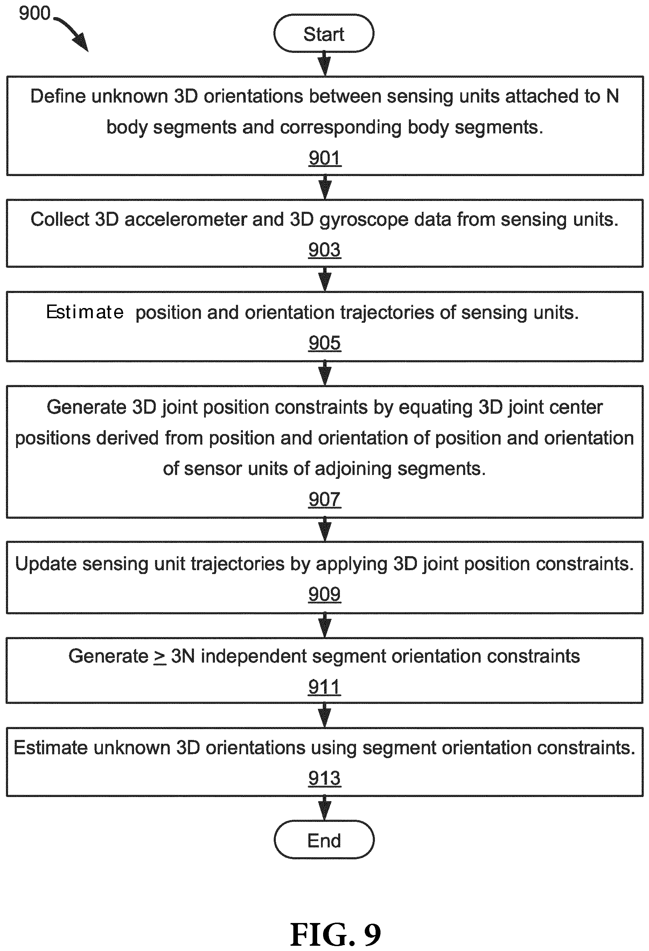

Continuing, the flowchart of Figure 9 illustrates a process 900 corresponding to an exemplary implementation of the described method. At stage 901 of the process 900, the processor 311 defines unknown 3D orientations between sensing units attached to N body segments and their corresponding body segments. At stage 903, the processor 311 collects 3D accelerometer and 3D gyroscope data from the sensing units.

The position and orientation trajectories of the sensing units are estimated by the processor 311 at stage 905, and 3D joint position constraints are generated by equating the 3D joint center positions derived from the position and orientation of two sensing units attached to adjoining segments at stage 907. The sensing unit trajectories are updated at stage 909 by applying the 3D joint position constraints.

Subsequently the processor 311 generates a set of at least 3N independent segment orientation constraints at stage 911, each constraint being a scalar function operating on a 3D orientation of a segment at one or more time instants. Finally, the processor 311 estimates the unknown 3D orientations using the segment orientation constraints at stage 913.

As noted above, those of skill in the art will appreciate that the described techniques can be straightforwardly extended to include other constraints, e.g., a known placement of the sensing units based on use of a suit that constrains sensing unit placement.

It will be appreciated that in view of the many possible embodiments to which the principles of the present disclosure may be applied, it should be recognized that the embodiments described herein with respect to the drawing figures are meant to be illustrative only and should not be taken as limiting the scope of the claims. Therefore, the techniques as described herein contemplate all such embodiments as may come within the scope of the following claims.

**Claims**

1. A method of inertial motion capture calibration with respect to a subject (200) having N segments connected by joints, wherein N>1 and each segment has affixed at least one sensing unit (301, 303, 305, 307), each sensing unit containing at least a 3D gyroscope and a 3D accelerometer, the method comprising:

    defining (901) unknown 3D orientations between sensing units and the corresponding segments the sensing units (301, 303, 305, 307) are attached to;
    collecting (903) 3D accelerometer and 3D gyroscope data from the sensing units (301, 303, 305, 307);
    estimating 3D position and 3D orientation trajectories of the sensing units by integration of the 3D accelerometer and 3D gyroscope data;
    deriving 3D joint center positions from the estimated 3D position and 3D orientation trajectories of the sensing units (301, 303, 305, 307);
    generating (907) 3D joint position constraints by equating pairs of 3D joint center positions derived from sensing units (301, 303, 305, 307) on adjoining segments;
    updating (909) the sensing unit trajectories by applying the 3D joint position constraints;
    **characterized by**:

        generating (911) a set of at least 3N independent segment orientation constraints, each constraint being a scalar function operating on a 3D orientation of a segment at one or more time instants; and
        estimating (913) the unknown 3D orientations by applying the segment orientation constraints.

2. The method in accordance with claim 1, wherein the subject (200) is a human body or part of a human body.

3. The method in accordance with claim 1, wherein the subject (200) comprises at least one of an object and a mechanical structure.

4. The method in accordance with any of the preceding claims, wherein the joints include at least one of hinge joints and ball-and-socket joints.

5. The method in accordance with any of the preceding claims, wherein the 3D accelerometer and 3D gyroscope data comprise orientation and velocity increment signals obtained from pre-processing with strap down integration, SDI.

6. The method in accordance with any of the preceding claims, wherein any of the sensing units (301, 303, 305, 307) further include a 3D magnetometer.

7. The method according to any of the preceding claims, wherein each segment orientation constraint is expressed as

$$f(R_t) = 0,$$

where the function $f: SO_3^n \to \mathbb{R}^1$ operates on a 3D segment orientation R at n time instants t relative to another frame, wherein this other frame can be any of an external reference frame, a sensor frame, or another segment frame

8. The method in accordance with any of the preceding claims, wherein the 3D orientation of segments in the segment orientation constraints are relative to any of an external reference frame, a sensor frame, or another segment frame.

9. The method in accordance with any of the preceding claims, wherein estimating the unknown 3D orientations further includes using additional known constraints.

10. A method according to any of the preceding claims, the method further comprising:

    defining (401) unknown 3D position vectors from the sensing units (301, 303, 305, 307) to corresponding joint centers;
    generating (407) 3D joint position constraints by equating 3D joint center positions derived from the 3D position and 3D orientation trajectories of two sensing units (301, 303, 305, 307) attached to adjoining segments and the unknown 3D position vectors; and
    estimating (409) the unknown 3D position vectors and the sensing unit trajectories by applying the 3D joint position constraints.

**11.** The method in accordance with claim 10, wherein estimating (409) the unknown 3D position vectors and the sensing unit trajectories further includes using additional known constraints on the 3D position vectors.

**12.** A system of inertial motion capture calibration with respect to a subject (200) having N segments connected by joints, wherein N>1, the system comprising:

at least one sensing unit affixed to each segment, each sensing unit containing at least a 3D gyroscope and a 3D accelerometer; and
a controller configured to:

define (901) unknown 3D orientations between sensing units (301, 303, 305, 307) and the corresponding segments the sensing units are attached to;
collect (903) 3D accelerometer and 3D gyroscope data from the sensing units (301, 303, 305, 307);
estimate 3D position and 3D orientation trajectories of the sensing units by integration of the 3D accelerometer and 3D gyroscope data
derive 3D joint center positions from the estimated 3D position and 3D orientation trajectories of the sensing units (301, 303, 305, 307);
generate (907) 3D joint position constraints by equating pairs of 3D joint center positions derived from sensing units (301, 303, 305, 307) on adjoining segments;
update (909) the sensing unit trajectories by applying the 3D joint position constraints; **characterized in that** the system is further configured to:

generate (911) a set of at least 3N independent segment orientation constraints, each constraint being a scalar function operating on a 3D orientation of a segment at one or more time instants; and
estimate (913) the unknown 3D orientations by applying the segment orientation constraints.

**13.** The system in accordance with claim 12, wherein the subject (200) is part of a human body.

**14.** The system in accordance with claim 12, wherein the subject (200) comprises at least one of an object and a mechanical structure.

**15.** The system in accordance with any of claims 12-14, wherein the joints include at least one of hinge joints and ball-and-socket joints

**16.** The system in accordance with any of claims 12-15, wherein the 3D accelerometer and 3D gyroscope data comprise orientation and velocity increment signals obtained from pre-processing with strap down integration, SDI.

**17.** The system in accordance with any of claims 12-16, wherein any of the sensing units (301, 303, 305, 307) further include a 3D magnetometer.

**18.** The system in accordance with any of claims 12-17, wherein the segment orientation constraints are formulated using a segment orientation relative to any of an external reference frame, a sensor frame, or another segment frame.

**19.** A non-transitory computer-readable medium having stored thereon computer-executable instructions for performing inertial motion capture calibration with respect to a subject (200) having N segments connected by joints, wherein N>1, with at least one sensing unit (301, 303, 305, 307) affixed to each segment, each sensing unit containing at least a 3D gyroscope and a 3D accelerometer, the computer-executable instructions comprising:

defining (901) unknown 3D orientations between sensing units (301, 303, 305, 307) and the corresponding segments to which the sensing units are attached;
collecting (903) 3D accelerometer and 3D gyroscope data from the sensing units (301, 303, 305, 307);
estimating 3D position and 3D orientation trajectories of the sensing units by integration of the 3D accelerometer and 3D gyroscope data
deriving 3D joint center positions from the estimated 3D position and 3D orientation trajectories of the sensing units (301, 303, 305, 307);
generating 3D joint position constraints (907) by equating pairs of 3D joint center positions derived from sensing units (301, 303, 305, 307) on adjoining segments;
updating (909) the sensing unit trajectories by applying the 3D joint position constraints; **characterized by**:

generating (911) a set of at least 3N independent segment orientation constraints, each constraint being a scalar function operating on a 3D orientation of a segment at one or more time instants; and

estimating (913) the unknown 3D orientations by applying the segment orientation constraints.

**20.** The non-transitory computer-readable medium in accordance with claim 19, wherein the subject includes part of a human body.

**21.** The non-transitory computer-readable medium in accordance with claim 19, wherein the subject comprises at least one of an object and a mechanical structure.

**22.** The non-transitory computer-readable medium in accordance with any of claims 19-21, wherein the joints include at least one of hinge joints and ball-and-socket joints.

**23.** The non-transitory computer-readable medium in accordance with any of claims 19-22, wherein the 3D accelerometer and 3D gyroscope data comprises orientation and velocity increment signals obtained from pre-processing with strap down integration, SDI.

**24.** The non-transitory computer-readable medium in accordance with any of claims 19-23, wherein any of the sensing units (301, 303, 305, 307) further includes a 3D magnetometer.

**25.** The non-transitory computer-readable medium in accordance with any of claims 19-24, wherein the instructions for generating a set of at least 3N independent segment orientation constraints comprise instructions for generating the set of at least 3N independent segment orientation constraints using a segment orientation relative to any of an external reference frame, a sensor frame, or another segment frame.


**Patentansprüche**

**1.** Ein Verfahren zur Trägheitsbewegungserfassungskalibrierung in Bezug auf ein Subjekt (200) mit N Segmenten, die durch Gelenke verbunden sind, wobei N>1 und jedes Segment mindestens eine Sensoreinheit (301, 303, 305, 307) befestigt hat, wobei jede Sensoreinheit mindestens ein 3D-Gyroskop und ein 3D-Beschleunigungsmesser enthält, wobei das Verfahren Folgendes umfasst:

Definieren (901) unbekannter 3D-Ausrichtungen zwischen Sensoreinheiten und den entsprechenden Segmenten, an denen die Sensoreinheiten (301, 303, 305, 307) angebracht sind;
Erfassen (903) von 3D-Beschleunigungsmesser- und 3D-Gyroskopdaten von den Sensoreinheiten (301, 303, 305, 307);
Schätzen von 3D-Positions- und 3D-Ausrichtungstrajektorien der Sensoreinheiten durch Integration der 3D-Beschleunigungsmesser- und 3D-Gyroskopdaten, wobei 3D-Gelenkmittelpositionen aus den geschätzten 3D-Positions- und 3D-Ausrichtungstrajektorien der Sensoreinheiten (301, 303, 305, 307) abgeleitet werden;
Erzeugen (907) von 3D-Gelenkpositionsbeschränkungen durch Gleichsetzen von Paaren von 3D-Gelenkmittelpositionen, die von Sensoreinheiten (301, 303, 305, 307) an benachbarten Segmenten abgeleitet werden;
Aktualisieren (909) der Trajektorien der Sensoreinheit durch Anwenden der 3D-Gelenkpositionsbeschränkungen;
**gekennzeichnet durch**: Erzeugen (911) eines Satzes von mindestens 3N unabhängigen Segmentausrichtungsbeschränkungen, wobei jede Beschränkung eine Skalarfunktion ist, die an einer 3D-Ausrichtung eines Segments zu einem oder mehreren Zeitpunkten arbeitet; und
Schätzen (913) der unbekannten 3D-Ausrichtungen durch Anwenden der Segmentausrichtungsbeschränkungen.

**2.** Verfahren nach Anspruch 1, wobei das Subjekt (200) ein menschlicher Körper oder ein Teil eines menschlichen Körpers ist.

**3.** Verfahren nach Anspruch 1, wobei das Subjekt (200) ein Objekt und/oder eine mechanische Struktur umfasst.

**4.** Verfahren nach einem der vorhergehenden Ansprüche, wobei die Gelenke mindestens eines von Drehgelenken und Kugelgelenken umfassen.

**5.** Verfahren nach einem der vorhergehenden Ansprüche, wobei die 3D-Beschleunigungsmesser- und 3D-Gyroskopdaten Ausrichtungs- und Geschwindigkeitsinkrementsignale umfassen, die aus einer Vorverarbeitung mit Strap-Down-Integration, SDI, erhalten werden.

**6.** Verfahren nach einem der vorhergehenden Ansprüche, wobei eine der Sensoreinheiten (301, 303, 305, 307) weiter ein 3D-Magnetometer enthält.

**7.** Verfahren nach einem der vorhergehenden Ansprüche, wobei jede Segmentausrichtungsbeschränkung ausgedrückt wird als

$$\mathrm{f}(\mathrm{R}_t) = 0,$$

wo die Funktion $\mathrm{f} \colon \mathrm{SO}_3^n \to \mathbb{R}^1$ an einer 3D-Segmentorientierung R zu n Zeitpunkten t relativ zu einem anderen Rahmen arbeitet, wobei dieser andere Rahmen ein externer Referenzrahmen, ein Sensorrahmen oder ein anderer Segmentrahmen sein kann.

8. Verfahren nach einem der vorhergehenden Ansprüche, wobei die 3D-Ausrichtung von Segmenten in den Segmentausrichtungsbeschränkungen relativ zu einem externen Referenzrahmen, einem Sensorrahmen oder einem anderen Segmentrahmen ist.

9. Verfahren nach einem der vorhergehenden Ansprüche, wobei das Schätzen der unbekannten 3D-Ausrichtungen weiter die Verwendung zusätzlicher bekannter Beschränkungen umfasst.

10. Verfahren nach einem der vorhergehenden Ansprüche, wobei das Verfahren weiter Folgendes umfasst Definieren (401) unbekannter 3D-Positionsvektoren von den Sensoreinheiten (301, 303, 305, 307) zu entsprechenden Gelenkzentren; Erzeugen (407) von 3D-Gelenkpositionsbeschränkungen durch Gleichsetzen von 3D-Gelenkmittelpunktpositionen, die aus den 3D-Positions- und 3D-Ausrichtungstrajektorien von zwei an benachbarten Segmenten angebrachten Sensoreinheiten (301, 303, 305, 307) und den unbekannten 3D-Positionsvektoren abgeleitet werden; und Schätzen (409) der unbekannten 3D-Positionsvektoren und der Trajektorien der Sensoreinheit durch Anwenden der 3D-Gelenkpositionsbeschränkungen.

11. Verfahren nach Anspruch 10, wobei das Schätzen (409) der unbekannten 3D-Positionsvektoren und der Trajektorien der Sensoreinheit weiter das Verwenden zusätzlicher bekannter Beschränkungen an den 3D-Positionsvektoren umfasst.

12. Ein System zur Trägheitsbewegungserfassungskalibrierung in Bezug auf ein Subjekt (200) mit N-Segmenten, die durch Gelenke verbunden sind, wobei N>1 ist, wobei das System Folgendes umfasst:

mindestens eine an jedem Segment angebrachte Sensoreinheit, wobei jede Sensoreinheit mindestens einen 3D-Gyroskop und einen 3D-Beschleunigungsmesser enthält; und eine Steuerung, die für konfiguriert ist, um:

unbekannte 3D-Ausrichtungen zwischen Sensoreinheiten (301, 303, 305, 307) und den entsprechenden Segmenten zu definieren, an denen die Sensoreinheiten angebracht sind; 3D-Beschleunigungsmesser- und 3D-Gyroskopdaten von den Sensoreinheiten (301, 303, 305, 307) zu erfassen (903); 3D-Positions- und der 3D-Ausrichtungstrajektorien der Sensoreinheiten durch Integration des 3D-Beschleunigungsmessers und der 3D-Gyroskopdaten zu schätzen 3D-Gelenkmittelpositionen aus den geschätzten 3D-Positions- und 3D-Ausrichtungstrajektorien der Sensoreinheiten (301, 303, 305, 307) abzuleiten; 3D-Gelenkpositionsbeschränkungen durch Gleichsetzen von Paaren von 3D-Gelenkmittelpositionen zu erzeugen (907), die von Sensoreinheiten (301, 303, 305, 307) an benachbarten Segmenten abgeleitet werden; Trajektorien der Sensoreinheit durch Anwenden der 3D-Gelenkpositionsbeschränkungen aktualisieren (909); **dadurch gekennzeichnet, dass** das System weiter konfiguriert ist, um:

einen Satz von mindestens 3N unabhängigen Segmentausrichtungsbeschränkungen zu erzeugen (911), wobei jede Beschränkung eine Skalarfunktion ist, die an einer 3D-Ausrichtung eines Segments zu einem oder mehreren Zeitpunkten arbeitet; und unbekannte 3D-Ausrichtungen durch Anwenden der Segmentausrichtungsbeschränkungen zu schätzen (913).

13. System nach Anspruch 12, wobei das Subjekt (200) Teil eines menschlichen Körpers ist.

14. System nach Anspruch 12, wobei das Subjekt (200) ein Objekt und/oder eine mechanische Struktur umfasst.

15. System nach einem der Ansprüche 12 bis 14, wobei die Gelenke mindestens eines von Drehgelenken und Kugelgelenken umfassen

16. System nach einem der Ansprüche 12 bis 15, wobei die 3D-Beschleunigungsmesser- und 3D-Gyroskop-Daten Ausrichtungs- und Geschwindigkeitsinkrementsignale umfassen, die aus einer Vorverarbeitung mit "Strap-Down-Integration", SDI, erhalten werden.

17. System nach einem der Ansprüche 12 bis 16, wobei eine der Sensoreinheiten (301, 303, 305, 307) weiter ein 3D-Magnetometer enthält.

18. System nach einem der Ansprüche 12 bis 17, wobei die Segmentausrichtungsbeschränkungen unter Verwendung einer Segmentausrichtung relativ zu einem externen Referenzrahmen, einem Sensorrahmen oder einem anderen Segmentrahmen formuliert werden.

**19.** Nichtflüchtiges computerlesbares Medium, auf dem computerausführbare Anweisungen gespeichert sind, um eine Trägheitsbewegungserfassungskalibrierung in Bezug auf ein Subjekt (200) mit N Segmenten durchzuführen, die durch Gelenke verbunden sind, wobei N>1 mit mindestens einer Sensoreinheit (301, 303, 305, 307) ist, die an jedem Segment angebracht sind, wobei jede Sensoreinheit mindestens ein 3D-Gyroskop und einen 3D-Beschleunigungsmesser enthält, wobei die computerausführbaren Anweisungen Folgendes umfassen:

Definieren (901) unbekannter 3D-Ausrichtungen zwischen Sensoreinheiten (301, 303, 305, 307) und den entsprechenden Segmenten, an denen die Sensoreinheiten befestigt sind;
Erfassen (903) von 3D-Beschleunigungsmesser- und 3D-Gyroskopdaten von den Sensoreinheiten (301, 303, 305, 307);
Schätzen der 3D-Positions- und der 3D-Ausrichtungstrajektorien der Sensoreinheiten durch Integration des 3D-Beschleunigungsmessers und der 3D-Gyroskopdaten
Ableiten von 3D-Gelenkmittelpositionen aus den geschätzten 3D-Positions- und 3D-Ausrichtungstrajektorien der Sensoreinheiten (301, 303, 305, 307);
Erzeugen von 3D-Gelenkpositionsbeschränkungen (907) durch Gleichsetzen von Paaren von 3D-Gelenkmittelpositionen, die von Sensoreinheiten (301, 303, 305, 307) an benachbarten Segmenten abgeleitet werden;
Aktualisieren (909) der Trajektorien der Sensoreinheit durch Anwenden der 3D-Gelenkpositionsbeschränkungen;
**gekennzeichnet durch**: Erzeugen (911) eines Satzes von mindestens 3N unabhängigen Segmentausrichtungsbeschränkungen, wobei jede Beschränkung eine Skalarfunktion ist, die an einer 3D-Ausrichtung eines Segments zu einem oder mehreren Zeitpunkten arbeitet; und
Schätzen (913) der unbekannten 3D-Ausrichtungen durch Anwenden der Segmentausrichtungsbeschränkungen.

**20.** Nichtflüchtiges computerlesbares Medium nach Anspruch 19, wobei das Subjekt einen Teil eines menschlichen Körpers umfasst.

**21.** Nichtflüchtiges computerlesbares Medium nach Anspruch 19, wobei das Subjekt ein Objekt und/oder eine mechanische Struktur umfasst.

**22.** Nichtflüchtiges computerlesbares Medium nach einem der Ansprüche 19 bis 21, wobei die Gelenke mindestens eines von Drehgelenken und Kugelgelenken umfassen.

**23.** Nichtflüchtiges computerlesbares Medium nach einem der Ansprüche 19 bis 22, wobei die 3D-Beschleunigungsmesser- und 3D-Gyroskopdaten Ausrichtungs- und Geschwindigkeitsinkrementsignale umfassen, die aus einer Vorverarbeitung mit "Strap-Down-Integration", SDI, erhalten werden.

**24.** Nichtflüchtiges computerlesbares Medium nach einem der Ansprüche 19 bis 23, wobei eine der Sensoreinheiten (301, 303, 305, 307) weiter ein 3D-Magnetometer enthält.

**25.** Nichtflüchtiges computerlesbares Medium nach einem der Ansprüche 19 bis 24, wobei die Anweisungen zum Erzeugen eines Satzes von mindestens 3N unabhängigen Segmentausrichtungsbeschränkungen Anweisungen zum Erzeugen des Satzes von mindestens 3N unabhängigen Segmentausrichtungsbeschränkungen umfassen, unter Verwendung einer Segmentausrichtung relativ zu einem externen Referenzrahmen, einem Sensorrahmen oder einem anderen Segmentrahmen.

**Revendications**

**1.** Procédé d'étalonnage de capture de mouvement inertiel par rapport à un sujet (200) ayant N segments reliés par des articulations, dans lequel N>1 et chaque segment est équipé d'au moins une unité de détection (301, 303, 305, 307), chaque unité de détection contenant au moins un gyroscope 3D et un accéléromètre 3D, le procédé comprenant :

la définition (901) d'orientations en 3D inconnues entre les unités de détection et les segments correspondants sur lesquels les unités de détection (301, 303, 305, 307) sont fixées ;
la collecte (903) de données d'accéléromètre 3D et de gyroscope 3D auprès des unités de détection (301, 303, 305, 307) ;
l'estimation de trajectoires de position 3D et d'orientation 3D des unités de détection par intégration des données d'accéléromètre 3D et de gyroscope 3D ;
la dérivation de positions centrales 3D des articulations à partir des trajectoires de position 3D et d'orientation 3D estimées des unités de détection (301, 303, 305, 307) ;
la génération (907) de contraintes de position 3D des articulations en mettant en équation des paires de positions centrales 3D de articulations dérivées des unités de détection (301, 303, 305, 307) sur des segments adjacents ;
la mise à jour (909) des trajectoires des unités de détection en appliquant les contraintes de position 3D des articulations ;
**caractérisé par** :

la génération (911) d'un ensemble d'au moins 3N contraintes d'orientation de segments indépendants, chaque contrainte étant une fonction scalaire qui fonctionne sur une orientation 3D d'un segment à un ou plusieurs moment(s) ; et

l'estimation (913) des orientations 3D inconnues en appliquant les contraintes d'orientation de segments.

2. Procédé selon la revendication 1, dans lequel le sujet (200) est un corps humain ou une partie d'un corps humain.

3. Procédé selon la revendication 1, dans lequel le sujet (200) comprend au moins l'un d'un objet et d'une structure mécanique.

4. Procédé selon l'une quelconque des revendications précédentes, dans lequel les articulations comprennent au moins l'une de articulations articulées et de articulations à rotule.

5. Procédé selon l'une quelconque des revendications précédentes, dans lequel les données d'accéléromètre 3D et de gyroscope 3D comprennent des signaux d'incrémentation d'orientation et de vitesse obtenus à partir d'un prétraitement avec intégration strapdown (à calcul d'incréments à partir d'une vitesse angulaire et une accélération linéaire), SDI.

6. Procédé selon l'une quelconque des revendications précédentes, dans lequel n'importe laquelle des unités de détection (301, 303, 305, 307) comprend en outre un magnétomètre 3D.

7. Procédé selon l'une quelconque des revendications précédentes, dans lequel chaque contrainte d'orientation de segment est exprimée comme suit :

$$f(R_t) = 0$$

où la fonction $f: SO_3^n \to \mathbb{R}^1$ fonctionne sur une orientation de segment 3D R à n moments par rapport à un autre repère, dans lequel cet autre repère peut être n'importe lequel parmi un repère de référence externe, un repère de capteur, ou un repère d'un autre segment.

8. Procédé selon l'une quelconque des revendications précédentes, dans lequel l'orientation 3D des segments dans les contraintes d'orientation des segments s'entend par rapport à n'importe lequel d'un repère de référence externe, d'un repère de capteur, ou d'un repère d'un autre segment.

9. Procédé selon l'une quelconque des revendications précédentes, dans lequel l'estimation des orientations 3D inconnues comprend en outre l'utilisation de contraintes inconnues supplémentaires.

10. Procédé selon l'une quelconque des revendications précédentes, le procédé comprenant en outre :

la définition (401) de vecteurs de position 3D inconnus entre les unités de détection (301, 303, 305, 307) et des centres d'articulation correspondants ;

la génération (407) de contraintes de position 3D d'articulations en mettant en équation les positions centrales 3D des articulations dérivées des trajectoires de position 3D et d'orientation 3D de deux unités de détection (301, 303, 305, 307) fixées sur des segments adjacents et les vecteurs de position 3D inconnus ; et

l'estimation (409) des vecteurs de position 3D inconnus et des trajectoires d'unités de détection en appliquant les contraintes de position 3D des articulations.

11. Procédé selon la revendication 10, dans lequel l'estimation (409) des vecteurs de position 3D inconnus et des trajectoires des unités de détection comprend en outre l'utilisation de contraintes connues supplémentaires sur les vecteurs de position 3D.

12. Système d'étalonnage de capture de mouvement inertiel par rapport à un sujet (200) ayant N segments reliés par des articulations, dans lequel N>1, le système comprenant :

au moins une unité de détection fixée sur chaque segment, chaque unité de détection contenant au moins un gyroscope 3D et un accéléromètre 3D ; et

un contrôleur configuré pour :

définir (901) des orientations 3D inconnues entre les unités de détection (301, 303, 305, 307) et les segments correspondants sur lesquels les unités de détection sont fixées ;

collecter (903) des données d'accéléromètre 3D et de gyroscope 3D auprès des unités de détection (301, 303, 305, 307) ;

estimer des trajectoires de position 3D et d'orientation 3D des unités de détection par intégration des données d'accéléromètre 3D et de gyroscope 3D ;

dériver des positions centrales 3D des articulations à partir des trajectoires de position 3D et d'orientation 3D estimées des unités de détection (301, 303, 305, 307) ;

générer (907) des contraintes de position 3D des articulations en mettant en équation des paires de positions centrales 3D d'articulations dérivées des unités de détection (301, 303, 305, 307) sur des segments adjacents ;

mettre à jour (909) les trajectoires des unités de détection en appliquant les contraintes de position 3D des articulations ;

**caractérisé en ce que** le système est en outre configuré pour :

générer (911) un ensemble d'au moins 3N contraintes d'orientation de segments indépendants, chaque contrainte étant une fonction scalaire qui fonctionne sur une orientation 3D d'un segment à un ou plusieurs moment(s) ; et estimer (913) les orientations 3D inconnues en appliquant les contraintes d'orientation de segments.

**13.** Système selon la revendication 12, dans lequel le sujet (200) fait partie d'un corps humain.

**14.** Système selon la revendication 12, dans lequel le sujet (200) comprend au moins l'un d'un objet et d'une structure mécanique.

**15.** Système selon l'une quelconque des revendications 12 à 14, dans lequel les articulations comprennent au moins l'une des articulations articulées et d'articulations à rotule.

**16.** Système selon l'une quelconque des revendications 12 à 15, dans lequel les données d'accéléromètre 3D et de gyroscope 3D comprennent des signaux d'incrémentation d'orientation et de vitesse obtenus à partir d'un prétraitement avec une intégration strapdown, SDI.

**17.** Système selon l'une quelconque des revendications 12 à 16, dans lequel n'importe laquelle des unités de détection (301, 303, 305, 307) comprend en outre un magnétomètre 3D.

**18.** Système l'une quelconque des revendications 12 à 17, dans lequel les contraintes d'orientation de segments sont formulées à l'aide d'une orientation de segments par rapport à n'importe lequel parmi un repère de référence externe, un repère de capteur, ou un repère d'un autre segment.

**19.** Support non transitoire lisible par un ordinateur stockant des instructions exécutables par un ordinateur qui permettent d'effectuer un étalonnage de capture de mouvement inertiel par rapport à un sujet (200) ayant N segments reliés par des articulations, dans lequel N>1, avec au moins une unité de détection (301, 303, 305, 307) fixée sur chaque segment, chaque unité de détection contenant au moins un gyroscope 3D et un accéléromètre 3D, les instructions exécutables par un ordinateur comprenant :

la définition (901) d'orientations 3D inconnues entre les unités de détection (301, 303, 305, 307) et les segments correspondants sur lesquels les unités de détection sont fixées ; la collecte (903) de données d'accéléromètre 3D et de gyroscope 3D auprès des unités de détection (301, 303, 305, 307) ; l'estimation de trajectoires de position 3D et d'orientation 3D des unités de détection par intégration des données d'accéléromètre 3D et de gyroscope 3D ; la dérivation de positions centrales 3D des articulations à partir des trajectoires de position 3D et d'orientation 3D estimées des unités de détection (301, 303, 305, 307) ; la génération (907) de contraintes de position 3D des articulations en mettant en équation des paires de positions centrales 3D d'articulations dérivées des unités de détection (301, 303, 305, 307) sur des segments adjacents ; la mise à jour (909) des trajectoires des unités de détection en appliquant les contraintes de position 3D des articulations ; **caractérisé par** :

la génération (911) d'un ensemble d'au moins 3N contraintes d'orientation de segments indépendants, chaque contrainte étant une fonction scalaire qui fonctionne sur une orientation 3D d'un segment à un ou plusieurs moment(s) ; et l'estimation (913) des orientations 3D inconnues en appliquant les contraintes d'orientation de segments.

**20.** Support non transitoire lisible par un ordinateur selon la revendication 19, dans lequel le sujet comprend des parties d'un corps humain.

**21.** Support non transitoire lisible par un ordinateur selon la revendication 19, dans lequel le sujet comprend au moins l'un d'un objet et d'une structure mécanique.

**22.** Support non transitoire lisible par un ordinateur selon l'une quelconque des revendications 19 à 21, dans lequel les articulations comprennent au moins l'un des articulations articulées et d'articulations à rotule.

**23.** Support non transitoire lisible par un ordinateur selon l'une quelconque des revendications 19 à 22, dans lequel les données d'accéléromètre 3D et de gyroscope 3D comprennent des signaux d'incrémentation d'orientation et de vitesse obtenus à partir d'un prétraitement avec intégration strapdown, SDI.

24. Support non transitoire lisible par un ordinateur selon l'une quelconque des revendications 19 à 23, dans lequel n'importe laquelle des unités de détection (301, 303, 305, 307) comprend en outre un magnétomètre 3D.

25. Support non transitoire lisible par un ordinateur selon l'une quelconque des revendications 19 à 24, dans lequel les instructions destinées à générer un ensemble d'au moins 3N contraintes d'orientation de segments indépendantes à l'aide d'une orientation des segments par rapport à n'importe lequel parmi un repère de référence externe, un repère de capteur, ou un repère d'un autre capteur.

**FIG. 1**

**FIG. 2**

FIG. 3

400

( Start )

Define unknown 3D position vectors from the sensor units to the corresponding joint centers.
401

Collect 3D accel. and 3D gyroscope data from sensor units.
403

Estimate position and orientation trajectories of sensor units.
405

Generate 3D joint position constraints by equating 3D joint center positions from position and orientation of sensor units of adjoining segments.
407

Estimate unknown 3D position vectors and sensor unit trajectories via 3D joint position constraints.
409

( End )

# FIG. 4

**FIG. 5**

**FIG. 6A**

**FIG. 6B**

**before sensor-to-segment calibration**

701

**after sensor-to-segment calibration**

703

Ex. 1
Ex. 2

Knee Flexion/Extension [deg]

Time [sec]

# FIG. 7

**FIG. 8A**

**FIG. 8B**

900

```
┌─────────────┐
│    Start    │
└─────────────┘
```

Define unknown 3D orientations between sensing units attached to N
body segments and corresponding body segments.
901

Collect 3D accelerometer and 3D gyroscope data from sensing units.
903

Estimate  position and orientation trajectories of sensing units.
905

Generate 3D joint position constraints by equating 3D joint center
positions derived from position and orientation of position and orientation
of sensor units of adjoining segments.
907

Update sensing unit trajectories by applying 3D joint position constraints.
909

Generate $\geq$ 3N independent segment orientation constraints
911

Estimate unknown 3D orientations using segment orientation constraints.
913

```
┌─────────────┐
│     End     │
└─────────────┘
```

## FIG. 9

**EP 3 064 134 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 7725279 B2
- US 2011109438 A1
- US 7725279 B